# EUROPEAN PATENT APPLICATION

(11) **EP 2 990 046 A1**
(43) Date of publication of application: **02.03.2016**
(21) Application number: 14788947.1
(22) Date of filing: 23.04.2014
(51) Int. Cl.: A61K 36/896, A61P 35/02

(54) **PHARMACEUTICAL COMPOSITION CONTAINING LIRIODENDRON TULIPIFERA L. EXTRACT FOR TREATING CHRONIC MYELOGENOUS LEUKEMIA**

(30) Priority: 26.04.2013 KR 20130046506
(71) Applicant: Cho Dang Pharm. Co., Ltd., Seoul 152-838 (KR)
(72) Inventor: KIM, Ki Woon, Seoul 138-797 (KR); YOO, Hye Dong, Seoul 152-838 (KR); HONG, Soon Sun, Seoul 122-891 (KR); KIM, Soo Jung, Ansan-si Gyeonggi-do 425-760 (KR); LEE, Hyun Seung, Incheon 406-738 (KR); FANG, Zhenghuan, Incheon 402-833 (KR); KANG, Su Jin, Jecheon-si Chungcheongbuk-do 390-100 (KR)
(74) Representative: D Young & Co LLP
(86) International application number: PCT/KR2014/003542
(87) International publication number: WO 2014/175652

(57) **Abstract**

The present invention relates to a pharmaceutical composition containing, as an active ingredient, a Liriodendron tulipifera L. bark extract for treating chronic myelogneous leukemia. More specifically, the present invention relates to the pharmaceutical composition, containing, as the active ingredient, the Liriodendron tulipifera L. bark extract useful in selectively inhibiting mutant enzyme T315I from a mutation of a BCR-ABL fusion gene that causes chronic myelogenous leukemia, and to a use of epi-Tulipinolide which is an active ingredient of the composition.

## Description

### Technical Field

The present invention relates to a pharmaceutical composition for treating chronic myelogenous leukemia containing the extract from the bark of Liriodendron tulipifera as active ingredient. More particularly, the present invention relates to a pharmaceutical composition containing the extract from the bark of Liriodendron tulipifera as active ingredient for selectively inhibiting the T315I enzyme variant produced from BCR-ABL fusion gene, as well as a pharmaceutical use of the extract from the bark of Liriodendron tulipifera containing epi-tulipinolide as active ingredient.

### Description of Prior Art

Liriodendron tulipifera L. is a kind of deciduous broad-leaved arboreal belonged to Magnoliaceae family. In the bark of Liriodendron tulipifera, alkaloid, sesquiterpene and/or lignan have been known to be included.

Further, in the extract of Liriodendron tulipifera, sesquiterpene lactone compounds including costunolide, tulipinolide, epi-tulipinolide, epi-tulipdienolide and/or gamma-liriodenolide have been included.

Further, parthenolide among these sesquiterpene lactones has been known as anti inflammatory and/or anti cancer agent mediating nuclear transcription factor NF-κB, p21 and/or cyclin D1. Particularly, parthenolide and/or LC-1 (dimethylamino-parthenolide) has been known as anti cancer agent for acute myeloid leukemia (AML) inducing its apoptosis by suppressing the activity of nuclear transcription factor NF-κB.

Leukemia is a kind of blood cancers caused by abnormal multiplication of blood cells, especially, white blood cells. In the blood of leukemia patient, a numerous number of immature white blood cells have been present. Since the number of normal blood cells becomes severely reduced due to the abnormal multiplication of white blood cell, the fundamental function of blood cannot be achieved. Further, abnormal multiplication of white blood cells can cause the destruction of normal tissue as similar symptom of auto immune disease.

It is a form of chronic myeloid leukemia characterized by the increased and unregulated growth of predominantly myeloid cells in the bone marrow and the accumulation of these cells in the blood. Further, the kinds of leukemia can be classified into acute myeloid leukemia, chronic myeloid leukemia, acute lymphocytic leukemia (ALL) and chronic lymphocytic leukemia (CLL).

Further, in case of the chronic myeloid leukemia, the abnormal myeloid cells appearing to be matured cannot play their roles. Such chronic myeloid leukemia has been associated with the characteristic chromosomal translocation called the Philadelphia chromosome.

The chronic myeloid leukemia shows some symptomatic difference compared to other type of leukemia. Especially, the transition of symptom from chronic multiplication stage showing slow multiplication progress of abnormal myeloid cells to accelerated stage showing severe symptomatic progess can occur suddenly. On the other hand, the chronic multiplication stage can be sustained for 3-4 years. Therefore, the therapy for the chronic myeloid leukemia at chronic multiplication stage has to be focused to prevent the occurrence of transition of symptom from chronic stage to accelerated stage, rather than to make a complete recovery. Of course, the most of patients enduring the rapid transition cannot be easily cured.

The causes of chronic myeloid leukemia have been known as the formation of BCR-ABL fusion gene which has been called as Philadelphia chromosome wherein the part of 9th chromosome and the part of 22nd chromosome has been translocated. Such BCR-ABL fusion gene can induce the expression of fusion protein showing tyrosine kinase activity, which has been known as involving the p210 protein biosynthesis. Since the biosynthesized p210 protein can inhibit the apoptosis of myeloid progenitor cells, myeloid progenitor cells can be abnormally multiplied, which results in the myeloid leukemia

Imatinib (marketed as Gleevec) has been used as 1st generation therapeutics for treating myeloid leukemia, which inhibits tyrosine kinase of BCR-ABL protein. However, it has the problem that the patients having Imatinib resistance have appeared. Fortunately, the 2nd generation therapeutics, such as, Dasatinib, Nilotinib and Bosutinib have been developed, which can overcome the resistance of Imatinib. However, such 2nd generation therapeutics cannot show the sufficient efficacy any longer, due to the occurrence of T315I enzyme variant expressed by mutant gene of BCR-ABL. Therefore, the 3rd generation therapeutics overcoming the handicaps of 1st and 2nd generation therapeutics has been required for effective treatment of chronic myeloid leukemia caused by T315I enzyme variant.

For this purpose, the inventors of present application have isolated the components having pharmacological activity from the bark of Liriodendron tulipifera including epi-tulipinolide and costunolide. Further, regarding each component from the bark of Liriodendron tulipifera, the experiments have been carried out to find out the pharmaceutical efficacy of said component against chronic myeloid leukemia.

Therefore, the inventors of present application have measured and confirmed that epi-tulipinolide from the extract of the bark of Liriodendron tulipifera is efficacious for treating chronic myeloid leukemia (CML) showing the resistance of both 1st generation therapeutics (Imatinib) and 2nd generation therapeutics. Finally, the present invention has been completed by developing a pharmaceutical composition containing epi-tulipinolide from the extract of the bark of Liriodendron tulipifera as active ingredient for treating chronic myeloid leukemia caused by T315I enzyme variant.

### Problem to be solved

The problem to be solved is to develop a pharmaceutical composition containing epi-tulipinolide from the extract of the bark of Liriodendron tulipifera efficacious for treating chronic myeloid leukemia (CML) showing the resistance of both 1st generation therapeutics (Imatinib) and 2nd generation therapeutics. Accordingly, the development of pharmaceutical composition containing epi-tulipinolide from the extract of the bark of Liriodendron tulipifera as active ingredient for treating chronic myeloid leukemia caused by T315I enzyme variant has been carried out.

### Means for solving the problem

The object of present application is to provide a pharmaceutical composition containing epi-tulipinolide as active ingredient from the extract of the bark of Liriodendron tulipifera for treating chronic myeloid leukemia (CML) by selectively inhibiting T315I enzyme variant expressed from BCR-ABL fusion gene.

Further, said pharmaceutical composition selectively inhibits the growth of Imatinib resistant chronic myeloid leukemia cells caused by T315I enzyme variant as well as induces the apoptosis of Imatinib resistance chronic myeloid leukemia cells by DNA fragmentation.

Further, said pharmaceutical composition further comprises the pharmaceutically acceptable carrier.

The another object of present application is to provide an oral preparation comprising said pharmaceutical composition, wherein said preparation is at least one selected from of tablet, pill, powder, hard capsule, gelatin banding hard capsule, soft capsule, chewing tablet in the form of caramel or jelly and/or oral solution.

Further, said oral preparation is selected from soft capsule or oral solution.

### Advantageous effect

The outstanding advantageous effect of present application is to provide a pharmaceutical composition containing epi-tulipinolide from the extract of the bark of Liriodendron tulipifera efficacious for treating chronic myeloid leukemia (CML) showing the resistance of both 1st generation therapeutics (Imatinib) and 2nd generation therapeutics. Accordingly, the pharmaceutical composition containing epi-tulipinolide from the extract of the bark of Liriodendron tulipifera as active ingredient can be used for treating chronic myeloid leukemia caused by T315I enzyme variant has been carried out.

### Brief description of drawings

Fig. 1 shows the cell viability of chronic myeloid leukemia cell lines, regarding the treatment of following 3 components, that are Imatinib as 1st generation therapeutics, CD-200 the extract of the bark of Liriodendron tulipifera which is useful for selective inhibition of T315I enzyme variant from BCR-ABL fusion gene and CD-EPT the composition including epi-tulipinolide as active ingredient. After treating above 3 components with chronic myeloid leukemia cell lines, XTT assay has been employed for measuring the cell viability.
Fig. 1a is the graph showing the cell viability as to Ba/F3 mother cell line, Ba/F3(BCR/ABL) cell line and Ba/F3(BCR/ABL-T315I) cell line, regarding Imatinib treatment depending on their concentrations.
Fig. 1b is the graph showing the cell viability as to Ba/F3 mother cell line, Ba/F3(BCR/ABL) cell line and Ba/F3(BCR/ABL-T315I) cell line, regarding CD-200 treatment depending on their concentrations.
Fig. 1c is the graph showing the cell viability as to Ba/F3 mother cell line, Ba/F3(BCR/ABL) cell line and Ba/F3(BCR/ABL-T315I) cell line, regarding CD-EPT treatment depending on their concentrations.
Fig. 2 shows the cell viability of chronic myeloid leukemia cell lines, regarding the treatment of following 3 components, that are Imatinib, CD-200 and CD-EPT. After treating above 3 components with chronic myeloid leukemia cell lines, XTT assay has been employed for measuring the IC₅₀ (inhibitory concentration 50).
Fig. 2a is the graph showing the cell viability as to Ba/F3 mother cell line, Ba/F3(BCR/ABL) cell line and Ba/F3(BCR/ABL-T315I) cell line, regarding Imatinib treatment for measuring IC₅₀.
Fig. 2b is the graph showing the cell viability as to Ba/F3 mother cell line, Ba/F3(BCR/ABL) cell line and Ba/F3(BCR/ABL-T315I) cell line, regarding CD-200 treatment for measuring IC₅₀.
Fig. 2c is the graph showing the cell viability as to Ba/F3 mother cell line, Ba/F3(BCR/ABL) cell line and Ba/F3(BCR/ABL-T315I) cell line, regarding CD-EPT treatment for measuring IC₅₀.
Fig. 3 shows the Western Blot photographs indicating if the treatment of each Imatinib, Dasatinib and CD-200 can selectively inhibit the autophosphorylation of BCR fusion protein or not, regarding wild type Ba/F3 cell line and Ba/F3/T315I cell line.
Fig. 4 shows the analysis result from the flow cytometry 24 hours after CD-200 treatment for measuring the change of cell cycle as to leukemia cell line.
Fig. 4a shows the result data from the flow cytometry of control group without treatment of CD-200. Fig. 4b shows the result data from the flow cytometry of 1µg/ml of CD-200 treatment. Fig. 4c shows the result data from the flow cytometry of 5µg/ml of CD-200 treatment.
Fig. 5 is the graphs showing the changed ratio of cell cycles after CD-200 treatment as shown in Fig. 4. The cell cycles have been measured by flow cytometry after Propidium iodide staining for 30 minutes.

### Preferred embodiment of invention

The object of the present invention is to provide a pharmaceutical composition containing the extract from the bark of Liriodendron tulipifera as active ingredient for selectively inhibiting the T315I enzyme variant produced from BCR-ABL fusion gene, as well as a pharmaceutical use of the extract from the bark of Liriodendron tulipifera containing epi-tulipinolide as active ingredient.

The other object of the present invention is to provide an oral preparation which is at least one formulation selected from of tablet, pill, powder, hard capsule, gelatin banding hard capsule, soft capsule, chewing tablet in the form of caramel or jelly and/or oral aqueous solution.

Further, the present invention is to provide a pharmaceutical composition containing epi-tulipinolide from the extract of the bark of Liriodendron tulipifera efficacious for treating chronic myeloid leukemia for selectively inhibiting the expression of T315I enzyme variant from BCR-ABL fusion gene. Further, said pharmaceutical composition also comprises at least one pharmaceutically acceptable carrier.

Chronic myeloid leukemia which can be effectively treated by the extract of the bark of Liriodendron tulipifera is a blood cancer characterized in uncontrolled abnormal multiplication of white blood cells. Further, in case of chronic myeloid leukemia, the multiplication of white blood cells is very slowly progressed, which results in the over expansion of distinguished matured cell ranges.

Further, said pharmaceutical composition further comprises Epitulipdienolide, Ridentin and/or Deacetyllipiferolide as another active ingredient.

The pharmaceutical composition of present invention can be administered in various routes, for example, oral, in the formulation of tablet, granule, capsule, powder, syrup, ointment or suppository, intravenous and/or intramuscular. The dose of pharmaceutical composition is 10~500mg per one day, which can be administered once or few times a day.

The present invention is also to provide an oral preparation containing said pharmaceutical composition, which is selected from of tablet, pill, powder, hard capsule, gelatin banding hard capsule, soft capsule, chewing tablet in the form of caramel or jelly and/or oral aqueous solution.

The pharmaceutical composition of present invention can include pharmaceutically acceptable carriers. For example, the additive, such as, acid, fatty acid and/or fatty alcohol can be included to improve the solubility of extract from bark of Liriodendron tulipifera as well as to improve the dissolution rate of extract from bark of Liriodendron tulipifera by increasing its dispersion. Further, the sugar component, such as, white sugar, maltose, purified white sugar, starch syrup can be included. Of course, any inorganic additive, such as, magnesium stearate, talc as well as diluent, such as, non-crystalline cellulose, calcium hydrogen phosphate, starch and/or mannitol can be included. Further, anti-oxidant, flavoring agent, preservative, flavor, sweetener, pigment, pH adjusting agent and/or viscosity adjusting agent can be included. The amount of this additive has been conventionally known.

As the acid, the fatty acid or the fatty alcohol in the pharmaceutical composition of present invention, citric acid, oleic acid, stearyl alcohol, myristic acid, linoleic acid, lauric acid, capric acid, caprylic acid and/or caproic acid can be used, but not be limited thereto.

As the antioxidant in the pharmaceutical composition of present invention, butylated hydroxy toluene, sodium bisulfite, α-tocopherol, vitamin C, β-carotene, tocopherol acetate, fumarate acid, nalic acid, butylated hydroxyanisole, propyl gallate and/or sodium ascorbate can be used, but not be limited thereto.

As the flavoring agent in the pharmaceutical composition of present invention, mixed fruit flavor, apple flavor, strawberry flavor, cherry flavor, mint, vanilla flavor, yogurt flavor, or drink flavor can be used, but not be limited thereto.

As the preservative in the pharmaceutical composition of present invention, benzoic acid, sodium benzoate, ethylparaben, methylparaben or propylparaben can be used, but not be limited thereto.

As the flavor in the pharmaceutical composition of present invention, menthol, peppermint oil, orange oil, clove oil, cinnamon oil, strawberry essence and/or other conventional plant extract or fruit aroma can be used, but not be limited thereto.

As the sweetener in the pharmaceutical composition of present invention, refined sugar, glucose, fructose, aspartame, stevioside, sorbitol, mannitol, oligosaccharide and/or starch syrup can be used, but not be limited thereto.

As the pigment in the pharmaceutical composition of present invention, Green No. 3, Red No. 2, Red No. 3, Blue No. 1, Blue No. 2, Yellow No. 4, Yellow No. 5, soluble mannitol, caramel, titanium oxide or ferric oxide can be used, but not be limited thereto.

As the pH adjusting agent in the pharmaceutical composition of present invention, sodium carbonate, sodium hydroxide, potassium hydroxide, triethanolamine or monoethanolamine can be used, but not be limited thereto.

As the viscosity adjusting agent in the pharmaceutical composition of present invention, hydroxypropyl cellulose (HPC), hydroxypropylmethylcellulose (HPMC), hydroxyethyl cellulose, ethyl cellulose, methyl cellulose, carboxymethyl cellulose, acacia, bentonite, alginic acid, propylene glycol alginate, polyvinylpyrrolidone, polyvinyl alcohol, carbopol, polycarbopil, tragacanth or xanthan can be used, but not be limited thereto.

The pharmaceutical composition of present invention can further comprise at least one selected from pharmaceutically acceptable carrier, additive and/or diluent.

The kinds of pharmaceutically acceptable carriers, additives and/or diluents can be at least one selected from lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, purified water, methyl hydroxybenzoate, propyl hydroxybenzoate, talc, magnesium stearate and/or mineral oil

The solid formulation for oral administration can be at least one selected from tablet, pill, powder, granule, hard capsule, soft capsule and/or chewing tablet in the form of caramel or jelly. Further, such solid formulation can also further comprise at least one diluents selected from starch, calcium carbonate, sucrose, lactose and/or gelatin. Further, the lubricant, such as magnesium stearate or talc can be included.

The liquid formulation for oral administration can be at least one selected from suspension, solution, emulsion and/or syrup. As simple diluents for liquid formulation, purified water and/or liquid type paraffin can be added. Further, wetting agent, sweetener, flavoring agent and/or preservative can be also included.

The formulation for non-oral administration can be at least one selected from sterilized solution, non-aqueous solution, emulsion, freeze-dried formulation and/or suppository. In the non-aqueous solution and suspension, propylene glycol, polyethylene glycol, plant oil including olive oil and/or injectable ester including ethyl oleate can be included. Further, the base of suppository can be at least one selected from Witepsol, macrogol, Tween 61, cacao oil, lauric oil and/or glycero gelatin.

The pharmaceutical composition extracted from the bark of Liriodendron tulipifera can be administered in various routes selected from oral, rectal, intravenous, intramuscular, subcutaneous, intrauterine epidural and/or cerebrovascular.

The present invention can be explained more concretely by following Preparation Examples and Examples. However, the scope of present invention cannot be limited by following Examples.

### (Preparation Example 1) Preparation of extract (CD-200) from bark of Liriodendron tulipifera

### 1. Solvent extraction step

200g of dried and chopped bark of 3-5 years old Liriodendron tulipifera has been agitated and extracted using 2000ml of ethyl acetate at room temperature for 24 hours. After concentrating and filtering the extracted mixture at reduced pressure, the preliminary extract has been obtained. The obtained preliminary extract has been further mixed and extracted using 500ml of butanol at room temperature for 2-4 hours. Then, the butanol soluble materials have been removed by separating and removing the butanol layer. Finally, 12.54g of crude extract has been obtained after concentrating the remained mixture.

### 2. Purification step

After dissolving 12.54g of obtained crude extract with 200ml of 70% ethanol, 200ml of n-hexane has been added and agitated to the crude extract mixture. After separating and removing the n-hexane layer, 70% ethanol layer has been obtained. Finally, 2.75g of purified extract has been obtained upon concentration and freeze drying the obtained mixture. This purified extract has been named as CD-200.

### (Preparation Example 2) Preparation and isolation of epi-tulipinolide component (CD-EPT) from bark of Liriodendron tulipifera as a main component of CD-200

The component of epi-tulipinolide has been isolated from the extract (CD-200) from bark of Liriodendron tulipifera using high performance liquid chromatography (HPLC) Alliance e2695 system by WATERS. The conditions for HPLC analysis are as follows. Reverse phase column Kromasil C18 has been used, 5mg/ml of sample has been loaded and 215nm of UV wave length (flow rate: 1.0ml/min) has been employed for measuring the amount of eluent. Further, the mobile phase gradient (water and methanol) condition has been employed. Finally, the component of epi-tulipinolide has been isolated from the fraction at retention time =~31 minutes. The extract has been named as CD-EPT.

### (Preparation Example 3) Isolation and identification of epi-tulipinolide (CD-EPT)

The identification of epi-tulipinolide in the obtained fraction has been measured by the comparison of retention time, after loading and passing both sample of fraction and sample of standard epi-tulipinolide through the HPLC column. Then, it has been confirmed that the components of obtained fraction chiefly consist of epi-tulipinolide, since both retention times of obtained fraction and epi-tulipinolide have been measured to be same. Further, the specific rotation of epitulipinolide has been measured. The measured value of epi-tulipinolide ([α]D=+74) from CD-200 has almost corresponded with reported value of epi-tulipinolide ([α]D=+76). Therefore, the presence of epitulipinolide has been confirmed by stereochemistry.

### (Example 1) Test for inhibiting the multiplication of leukemia cell line by CD-200 and CD-EPT

To find out if CD-200 and CD-EPT can selectively inhibit the T315I enzyme variant from BCR-ABL fusion gene in chronic myeloid leukemia, the test for inhibiting the multiplication of leukemia cell line by the extract of the bark of Liriodendron tulipifera (CD-200) and the component of epi-tulipinolide (CD-EPT) has been carried out.

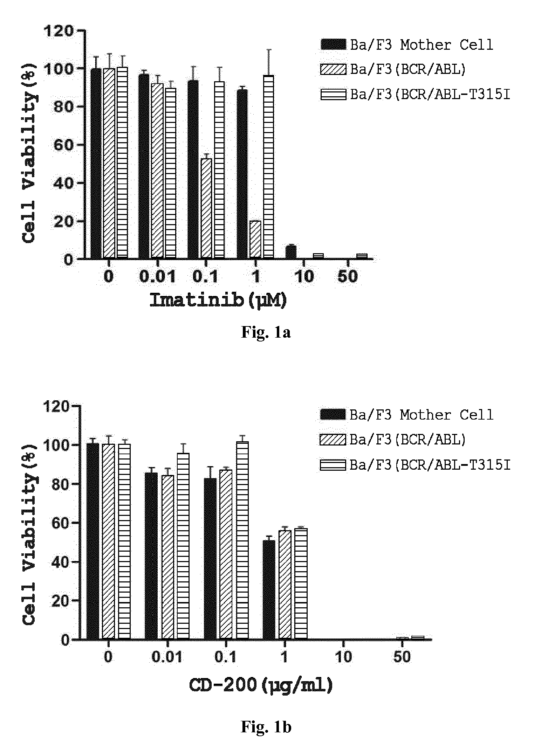

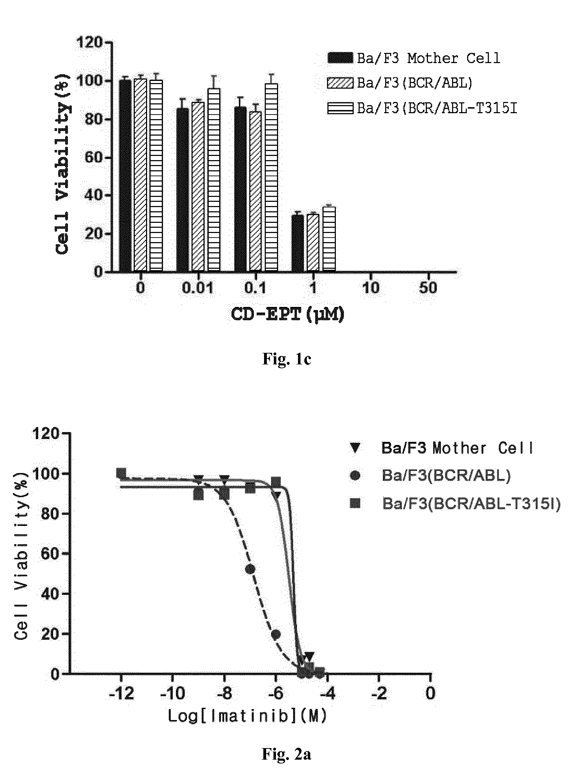

For measuring the cell viability indicating the inhibition of multiplication, in vitro XTT assay has been employed. Specifically, after loading 1x10³ of Ba/F3 cells which are leukemia cell lines containing BCR-ABL fusion gene into 96 well plate, 0.01∼50µM of 1st generation therapeutics for chronic myeloid leukemia (Imatinib), 0.01∼50µg/ml of the extract of the bark of Liriodendron tulipifera (CD-200) and 0.01∼50µM of the component of epi-tulipinolide (CD-EPT) have been added and injected to the plate. To find out the selective inhibition of T315I enzyme variant from BCR-ABL fusion gene, the cell lines have been incubated for 48 hours at 37°C. After incubation, the sample has been treated with XTT formazan (WEL GENE) for 4 hours at 37°C. Finally, the cell viability has been measured using ELISA through the absorbance at 620nm, 540nm (Fig.1).

As shown in Fig.1, in case of treating the cell lines with 1µM of Imatinib, 1µg/ml of CD-200 and 1µM of CD-EPT respectively for measuring the cell viability, the treatment of CD-200 and CD-EPT can effectively inhibit the cell growth in respect to Ba/F3 mother cell line and Ba/F3 (BCR/ABL-T315I) cell line. However, the treatment of CD-200 and CD-EPT cannot significantly inhibit the cell growth in respect to Ba/F3(BCR/ABL) cell line. Further, the treatment of CD-200 and CD-EPT can show the effective inhibition of cell growth compared to Imatinib in respect to Ba/F3 (BCR/ABL-T315I) cell line (Fig. 1).

### (Example 2) Test for measuring the inhibition concentration by CD-200 and CD-EPT against multiplication of leukemia cell lines

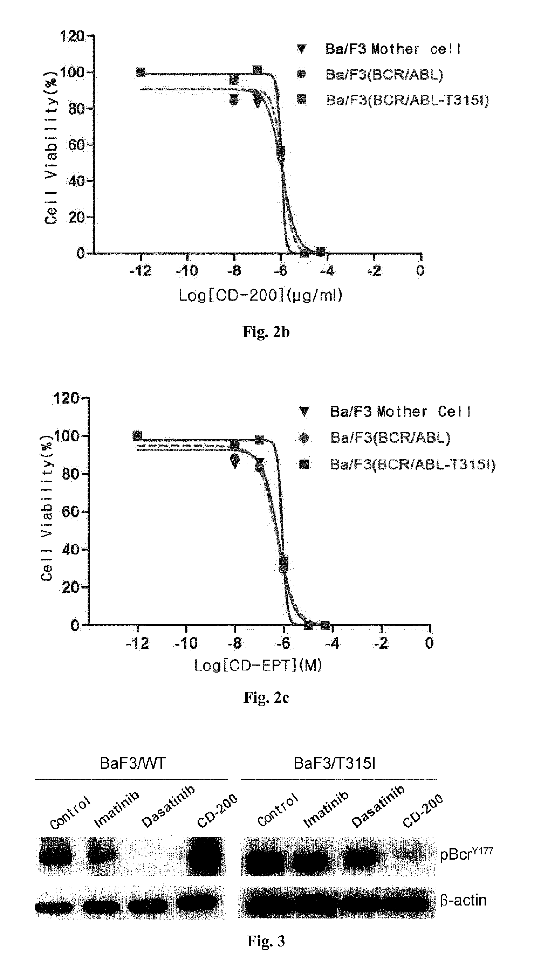

After measuring the cell viability of leukemia cell lines as to the treatment of Imatinib, CD-200 and CD-EPT as the same manner of Example 1, IC₅₀ has been measured against each leukemia cell line. The result has been shown in Fig. 2.

Regarding Ba/F3 (BCR/ABL-T315I) mutant, IC₅₀ values have been measured as follows. 1µg/ml of CD-200, 0.8µM of CD-EPT and 5µM of Imatinib. Therefore, the treatment of CD-200 and CD-EPT shows 5 times effective inhibition capability compared to Imatinib (Fig. 2). Therefore, CD-200 and CD-EPT of present invention show strong in vitro inhibition capability against the multiplication of leukemia cell lines, especially Ba/F3 (BCR/ABL-T315I) mutant.

### (Example 3) Western Blot test for confirming the selective inhibition of expression of BCR-ABL fusion gene by CD-200

To find out the selective inhibition of expression of BCR-ABL fusion gene by CD-200, Western Blot test has been carried out. Specifically, 1x10⁶ cells of wild type of BaF3 (BaF3/WT) and 1x10⁶ cells of BaF3/T315I which is leukemia cell line expressing BCR-ABL fusion gene have been loaded into the 10cm of dish. After treating the leukemia cell lines with 1µg/ml of CD-200, Imatinib and Dasatinib, which are therapeutics for chronic myeloid leukemia, cell lines have been incubated for 1 hour at 37°C. After incubation, cultured cells have been made into cell lysis. Finally, it has been measured if it is inhibited by autophosphorylation of BCR in the level of protein (Fig. 3).

As shown in Fig. 3, it has been confirmed that CD-200 shows the effective inhibition of autophosphorylation of BCR in the BaF3/T315I mutant compared to conventional therapeutics of Imatinib. Therefore, CD-200 has been confirmed as useful therapeutics for selectively inhibiting the multiplication of BCR-ABL/T315I mutant compared to conventional therapeutics.

### (Example 4) Test for confirming the change of cell cycle by CD-200

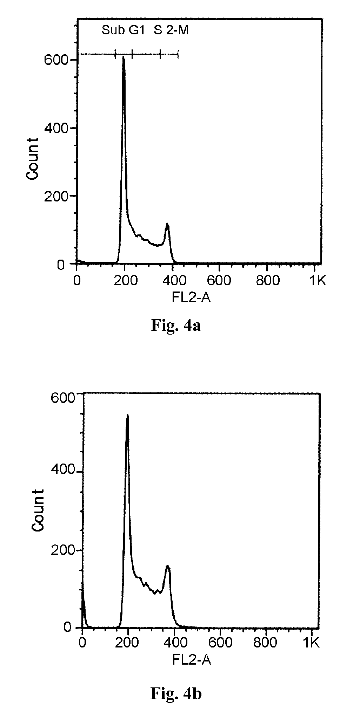

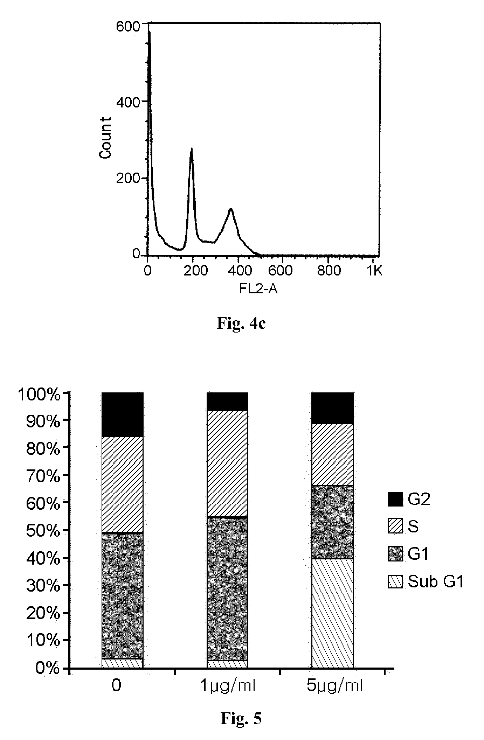

The cell cycle regarding leukemia cell line has been measured to find out if the cell cycle has been changed by the treatment of CD-200 affecting the cell viability. Specifically, after loading 2x10⁵ cells of BaF3/T315I which is leukemia cell line expressing BCR-ABL fusion gene into 10cm of dish, 0 (none), 1µg/ml and 5µg/ml of CD-200 have been and treated and incubated with cell lines for 24 hours at 37 °C. After incubation, cultured cells have been separated using centrifuge. Then, cells have been fixed with 75% of ethanol for 12 hours at - 20°C. After staining with Propidium iodide (PI) for more than 30 minutes, flow cytometry has been measured. Finally, after confirming the change of cell cycle, the ratio of cell cycle has been confirmed and illustrated by graphs (Fig. 4, Fig. 5).

As shown in Fig. 4 and Fig. 5, the treatment of CD-200 has induced the apoptosis of BaF3/T315I in a dose dependent manner compared to non-treatment of CD-200. Accordingly, prior to G1 phase, the peak has appeared. From this measurement, it has been confirmed that CD-200 induces the apoptosis. The ratio of apoptosis of the cells without CD-200 treatment shows 3%, while the ratio of apoptosis of the cells with 5µg/ml CD-200 treatment shows 38%. Therefore, it has been confirmed that CD-200 treatment can suspend the G1 phase in the cell cycle as well as reduce the cell viability of BaF3/T315I mutant.

## Claims

1. A pharmaceutical composition containing epi-tulipinolide as active ingredient from the extract of the bark of Liriodendron tulipifera for treating chronic myeloid leukemia (CML) by selectively inhibiting T315I enzyme variant expressed from BCR-ABL fusion gene.

2. The pharmaceutical composition according to claim 1, wherein said composition selectively inhibits the growth of Imatinib resistant chronic myeloid leukemia cells caused by T315I enzyme variant as well as induces the apoptosis of Imatinib resistance chronic myeloid leukemia cells by DNA fragmentation.

3. The pharmaceutical composition according to claim 1, further comprises the pharmaceutically acceptable carrier.

4. An oral preparation comprising the pharmaceutical composition of claim 3, wherein said preparation is at least one selected from of tablet, pill, powder, hard capsule, gelatin banding hard capsule, soft capsule, chewing tablet in the form of caramel or jelly and/or oral solution.

5. The oral preparation according to claim 4, wherein said preparation is selected from soft capsule or oral solution.
